# EUROPEAN PATENT APPLICATION

(11) **EP 1 813 601 A1**
(43) Date of publication of application: **01.08.2007**
(21) Application number: 05799246.3
(22) Date of filing: 28.10.2005
(51) Int. Cl.: C07C 309/82, C07C 303/02

(54) **METHOD FOR PRODUCING FLUORINE-CONTAINING FLUOROSULFONYL ALKYLVINYL ETHER**

(30) Priority: 15.11.2004 JP 2004330195
(71) Applicant: DAIKIN INDUSTRIES, LTD., Osaka-shi, Osaka 530-8323 (JP)
(72) Inventor: SUGIYAMA, Akinari c/o Yodogawa Seisakusho,, Settsu-shi, Osaka 5660044 (JP); ICHIHARA, Kazuyoshi c/o Yodogawa Seisakusho,, Settsu-shi, Osaka 5660044 (JP); SHINOKI, Noriyuki c/o Yodogawa Seisakusho,, Settsu-shi, Osaka 5660044 (JP); MANTANI, Toshiya c/o Yodogawa Seisakusho,, Settsu-shi, Osaka 5660044 (JP); KONDOU, Masahiro c/o Yodogawa Seisakusho,, Settsu-shi, Osaka 5660044 (JP)
(74) Representative: Luderschmidt, Schüler & Partner
(86) International application number: PCT/JP2005/019874
(87) International publication number: WO 2006/051697

(57) **Abstract**

The present invention provides a method of producing a fluorine-containing fluorosulfonylalkyl vinyl ether represented by the formula CF₂=CFOCF₂CF₂SO₂F, the method comprising reacting a fluorine-containing chlorosulfonylalkyl vinyl ether represented by the formula CF₂=CFOCF₂CF₂SO₂Cl with a fluorinating agent represented by the formula KF·(HF)ₙ (wherein n is 0 to 5) in a polar organic solvent at a temperature not higher than 70°C.

The method of the invention produces the fluorine-containing fluorosulfonylalkyl vinyl ether in high yield at low cost in a simple, industrially advantageous manner.

## Description

### TECHNICAL FIELD

The present invention relates to a method of producing a fluorine-containing fluorosulfonylalkyl vinyl ether.

### BACKGROUND ART

The fluorine-containing fluorosulfonylalkyl vinyl ether represented by the formula CF₂=CFOCF₂CF₂SO₂F is useful as an industrial raw material such as a starting material for producing ion exchange membranes, etc.

For example, a known method for producing a fluorine-containing fluorosulfonylalkyl vinyl ether or other sulfonyl vinyl ethers comprises adding hexafluoropropylene oxide to FCOCF₂SO₂F and then thermally decomposing the obtained acid fluoride derivative (see Patent Document 1 below).

In the above known method, when two or more molecules of hexafluoropropylene oxide are added to FCOCF₂SO₂F, sulfonyl vinyl ethers represented by CF₂=CFO(CF₂CF(CF₃)O)ₙCF₂CF₂SO₂F can be obtained. However, when one molecule of hexafluoropropylene oxide is added, a cyclization product represented by the following formula: 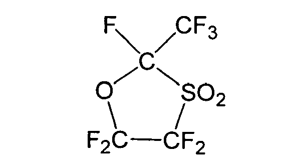 is obtained as a main product, with the result that fluorine-containing fluorosulfonylalkyl vinyl ethers represented by CF₂=CFOCF₂CF₂SO₂F can hardly be obtained.

Another known method comprises forming a cyclization product represented by the following formula: 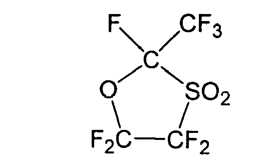 using FCOCF(CF₃)OCF₂CF₂SO₂F as a starting material, followed by ring opening using CH₃ONa to form CF₂=CFOCF₂CF₂SO₃Na, then chlorinating the terminal SO₃Na group to synthesize CF₂=CFOCF₂CF₂SO₂Cl and fluorinating the terminal SO₂Cl group to SO₂F using sulfolane as a solvent and NaF as a fluorinating agent (see Patent Document 2 below).

However, according to the above known method, the CF₂=CFOCF₂CF₂SO₂Cl conversion ratio is about 70% and the fluoride selectivity is about 85%. Thus this method cannot produce the desired fluoride in a sufficient yield.

When the fluorination reaction is carried out under reflux in order to increase the conversion ratio from chloride, the product decomposes gradually. Therefore, the produced fluoride needs to be taken out immediately in order to obtain the fluoride in a good yield, thus requiring very complicated reaction control. This method has another problem in the very large amount of industrial waste due to the necessity of using NaF in an amount of about 5 equivalents per equivalent of CF₂=CFOCF₂CF₂SO₂Cl, thus being difficult to carry out on an industrial scale.
Patent Document 1: British Patent No. 1,034,197
Patent Document 2: U.S. Patent No. 3,560,568

### DISCLOSURE OF THE INVENTION

### PROBLEM TO BE SOLVED BY THE INVENTION

The present invention has been achieved in view of the above-mentioned state of the art. A principal object of the present invention is to provide a method of producing a fluorine-containing fluorosulfonylalkyl vinyl ether in high yield at low cost in a simple, industrially advantageous manner.

### MEANS FOR SOLVING THE PROBLEM

The present inventors conducted extensive research in order to solve the above-mentioned problem. As a result, the inventors found that when CF₂=CFOCF₂CF₂SO₂Cl is used as a starting material and reacted with KF·(HF)ₙ as a fluorinating agent under highly specific conditions, i.e., in a polar organic solvent at a low temperature not higher than about 70°C, a fluorine-containing fluorosulfonylalkyl vinyl ether can be obtained in high yield. The inventors have accomplished the present invention, based on this finding.

The present invention provides the following methods of producing a fluorine-containing fluorosulfonylalkyl vinyl ether.
1. A method of producing a fluorine-containing fluorosulfonylalkyl vinyl ether represented by the formula CF₂=CFOCF₂CF₂SO₂F, the method comprising reacting a fluorine-containing chlorosulfonylalkyl vinyl ether represented by the formula CF₂=CFOCF₂CF₂SO₂Cl with a fluorinating agent represented by the formula KF·(HF)ₙ (wherein n is 0 to 5) in a polar organic solvent at a temperature not higher than 70°C.
2. The method according to item 1 wherein the reaction temperature is 20°C to 65°C.
3. The method according to item 1 wherein the polar organic solvent is sulfolane or adiponitrile.

According to the method of producing a fluorine-containing fluorosulfonylalkyl vinyl ether of the invention, a known fluorine-containing chlorosulfonylalkyl vinyl ether represented by the formula CF₂=CFOCF₂CF₂SO₂Cl is used as a starting material and reacted with a fluorinating agent to form a fluorine-containing fluorosulfonylalkyl vinyl ether represented by CF₂=CFOCF₂CF₂SO₂F.

The starting fluorine-containing chlorosulfonylalkyl vinyl ether represented by the formula CF₂=CFOCF₂CF₂SO₂Cl can be obtained, for example, by the method described in U.S. Patent No. 3,560,568, i.e., by using a known compound represented by FCOCF(CF₃)OCF₂CF₂SO₂F as a starting material to form a cyclization product thereof, followed by ring opening using CH₃ONa to form CF₂=CFOCF₂CF₂SO₃Na and then chlorinating the terminal SO₃Na group. The florine-containing chlorosulfonylalkyl vinyl ether obtained by this method may be isolated and purified before use, or the crude reaction mixture obtained by the above reactions may be used as is.

The use of a polar organic solvent as a solvent is essentially required in the production method of the invention. The polar organic solvent used in the invention is not particularly limited, and examples of usable solvents include sulfolane, dimethylformamide (DMF), dimethylsulfoxide (DMSO), ethyl acetate, monoglyme, diglyme, triglyme, tetraglyme, adiponitrile, hexamethylphosphoroamide (HMPA), acetonitrile, propionitrile, tetrahydrofuran (THF), dioxane, methanol, ethanol, isopropyl alcohol (IPA), methyl ethyl ketone (MEK), methyl isobutyl ketone (MIBK), etc.

When a fluorine-containing chlorosulfonylalkylvinyl ether is reacted with a fluorinating agent in such a polar organic solvent, the desired fluorine-containing fluorosulfonylalkyl vinyl ether can be obtained with high conversion ratio and high selectivity. Furthermore, since the desired compound can be obtained in high yield, regardless of the material of the reaction vessel, any suitable reaction vessel can be used.

Of the above-mentioned polar organic solvents, preferable solvents are, for example, sulfolane, adiponitrile, monoglyme, diglyme, triglyme, tetraglyme, DMF, DMSO, etc.

Particularly preferable polar organic solvents are sulfolane, adiponitrile, etc., which have a little difference in boiling point from a fluorine-containing fluorosulfonylalkyl vinyl ether and from which the fluorine-containing fluorosulfonylalkylvinyl ether separates to form two phases. Sulfolane is especially preferable. When such a solvent is used, the solvent and the intended fluorine-containing fluorosulfonylalkylvinyl ether separate into two phases, so that the intended compound can be easily obtained by a liquid separation method, etc.

The amount of polar organic solvent used is not particularly limited and may be any amount that allows the fluorinating agent to be dissolved or dispersed uniformly. The polar organic solvent is usually used in an amount of about 10 to about 500 parts by weight per 100 parts by weight of the fluorine-containing chlorosulfonylalkyl vinyl ether.

The use of a compound represented by the formula KF·(HF)ₙ (wherein n is 0 to 5) as a fluorinating agent is essentially required in the production method of the invention. By using such a specific fluorinating agent, the intended fluorine-containing fluorosulfonylalkyl vinyl ether can be obtained in very high yield. In contrast, when NaF is used as the fluorinating agent, a low CF₂=CFOCF₂CF₂SO₂Cl conversion ratio and an unsatisfactory fluoride selectivity result.

KF is particularly preferable as the fluorinating agent due to its high fluorinating ability, low possibility of corroding the reaction vessel, etc.

The fluorinating agent is usually used in an amount of about 0.5 to about 10 moles, and more preferably about 1 to about 5 moles, per mole of the fluorine-containing chlorosulfonylalkyl vinyl ether.

In the production method of the invention, it is essentially required to react a fluorine-containing chlorosulfonylalkyl vinyl ether represented by the formula CF₂=CFOCF₂CF₂SO₂Cl with a fluorinating agent represented by the formula KF·(HF)ₙ (wherein n is 0 to 5) in a polar organic solvent at a temperature not higher than about 70°C. By carrying out the reaction under such highly specific conditions, the intended fluorine-containing fluorosulfonylalkyl vinyl ether can be obtained in high yield. In contrast, when the reaction is carried out at temperatures higher than about 70°C, a large amount of a cyclization product is generated and the fluorine-containing fluorosulfonylalkyl vinyl ether is obtained in a very low yield.

The minimum reaction temperature is not particularly limited, as long as it is higher than the melting point of the solved used. The reaction temperature is usually in the range of about -20°C to about 70°C, and preferably about 20°C to about 65°C.

Although the reaction may be carried out at reduced pressure, atmospheric pressure, or elevated pressure, atmospheric pressure is preferable.

The reaction time is usually about 0.5 to about 48 hours, and preferably about 1 to about 24 hours.

The fluorine-containing fluorosulfonylalkyl vinyl ether obtained by the invention can be recovered by a method such as distillation, liquid separation, etc. When the ether is recovered by distillation, vacuum distillation at a temperature not higher than about 70°C is preferably used to prevent generation of by-products. When the ether is recovered by liquid separation, the recovery may be performed after quenching with water. When the reaction is performed in a highly polar solvent such as sulfolane, adiponitrile, etc., two-phase separation occurs, so that the fluorine-containing fluorosulfonylalkyl vinyl ether can be recovered by liquid separation as is.

The fluorine-containing fluorosulfonylalkyl vinyl ether represented by the formula CF₂=CFOCF₂CF₂SO₂F can be obtained in good yield by the above method.

The obtained crude compound can be purified by a known method such as distillation, column chromatography, etc.

The fluorine-containing fluorosulfonylalkyl vinyl ether obtained in the above manner is useful as a monomer component for producing a polymer electrolyte for fuel cells, etc.

### EFFECT OF THE INVENTION

According to the method of the invention, a reaction is carried out using a specific fluorinating agent in a polar organic solvent at a low temperature not higher than about 70°C to suppress the generation of by-products, thereby producing the intended fluorine-containing fluorosulfonyl ether in high yield.

Therefore, the method of the invention can produce the intended fluorine-containing fluorosulfonyl ether in high yield in an industrially advantageous manner without requiring complicated operations.

### BEST MODE FOR CARRYING OUT THE INVENTION

The present invention is described in more detail with reference to the following Examples.

### Example 1

60.0 g (197.7 mmol) of 97.8 mass% CF₂=CFOCF₂CF₂SO₂Cl, 17.7 g (305.2 mmol) of KF, and 66.4 g of sulfolane were placed into a 300-ml SUS 316 autoclave, and the temperature was raised to 60°C. The reaction was allowed to proceed at 60°C for 3 hours while stirring at 200 rpm.

After completion of the reaction, two dry ice-acetone traps were connected to the outlet side of the autoclave, and a pressure retainer and a pump were attached to the outlet side of the dry ice-acetone traps.

Thereafter, the gas in the system was blown off to reduce the inner pressure to ambient pressure and then further to 240 mmHg. With the heating temperature set to 70°C, the liquid was heated by a heater and withdrawn from the system. The withdrawn liquid was analyzed by NMR.

After withdrawing the inner solution, a mixture of 100 ml of H₂O and 50 ml of CCl₄ was placed in the autoclave and stirred for 1 hour. The mixture was then subjected to liquid separation and the upper and lower layers were analyzed by NMR.

The analysis results show that the CF₂=CFOCF₂CF₂SO₂Cl conversion ratio was 99.6% and CF₂=CFOCF₂CF₂SO₂F was obtained in a yield of 91.5%.

### Example 2

A dropping funnel containing 10 g (33.0 mmol) of 97.8 mass% CF₂=CFOCF₂CF₂SO₂Cl was attached to a 100 ml four-necked flask containing 2.95 g (50.9 mmol) of KF and 50 ml of acetonitrile.

CF₂=CFOCF₂CF₂SO₂Cl was added dropwise to the flask at room temperature in such a manner that the reaction vessel temperature did not exceed 35°C. After the addition, the mixture was stirred for about 1 day to allow the reaction to proceed at 32°C to 34°C. After the reaction, the reaction mixture was quenched with water and subjected to liquid separation and the lower layer was withdrawn. The withdrawn liquid was analyzed by ¹⁹F-NMR. The analysis results show that the CF₂=CFOCF₂CF₂SO₂Cl conversion ratio was 99.8% and CF₂=CFOCF₂CF₂SO₂F was obtained in a yield of 92.7%.

### Comparative Example 1

21.3 g (507.1 mmol) of NaF and 30.0 g of sulfolane as a solvent were placed in a 100 ml four-necked flask. A Liebig condenser was then attached to the flask, and a device for withdrawing the product was incorporated. The product was trapped by dry ice-acetone cooling.

52.7 g (177.7 mmol) of CF₂=CFOCF₂CF₂SO₂Cl was placed in the flask and the reaction was allowed to proceed at 73°C while heating. The obtained product was analyzed by ¹⁹F-NMR. The analysis results show that the CF₂=CFOCF₂CF₂SO₂Cl conversion ratio was 68.8% and CF₂=CFOCF₂CF₂SO₂F was obtained in a yield of 51.4%.

## Claims

1. A method of producing a fluorine-containing fluorosulfonylalkyl vinyl ether represented by the formula: CF₂=CFOCF₂CF₂SO₂F, the method comprising reacting a fluorine-containing chlorosulfonylalkyl vinyl ether represented by the formula CF₂=CFOCF₂CF₂SO₂Cl with a fluorinating agent represented by the formula KF·(HF)ₙ (wherein n is 0 to 5) in a polar organic solvent at a temperature not higher than 70°C.

2. The method according to claim 1 wherein the reaction temperature is 20°C to 65°C.

3. The method according to claim 1 wherein the polar organic solvent is sulfolane or adiponitrile.
